# EUROPEAN PATENT APPLICATION

(11) **EP 1 844 752 A1**
(43) Date of publication of application: **17.10.2007**
(21) Application number: 06708828.6
(22) Date of filing: 13.01.2006
(51) Int. Cl.: A61J 1/16, B01L 9/06, B65D 77/04, B65D 81/18, F25D 3/00

(54) **DEVICE FOR THE TRANSPORT OF BIOLOGICAL SAMPLES AND SIMILAR**

(30) Priority: 02.02.2005 ES 200500188
(71) Applicant: Kern Frio, S.A., 08021 Barcelona (ES)
(72) Inventor: ORRIOLS SALLÉS, Juan Alberto, E-08009 Barcelona (ES); CARRÉ PEREZ, Andreu, E-08801 Mataró (ES)
(74) Representative: Durán Moya, Carlos
(86) International application number: PCT/ES2006/000009
(87) International publication number: WO 2006/082262

(57) **Abstract**

The invention relates to a device for the transport of biological samples and similar. The inventive device is of the type that comprises at least one container suitable for the introduction of samples, an outer casing and a system for closing said container(s). The space between the container(s) and the casing can be filled with a material that provides resistance to an increase in the temperature inside the containers owing to heat exchange with the environment. The invention is **characterised in that** the container(s) comprise parts that are independent of the outer casing, whereby each container and the outer casing are joined in a leak-tight manner at the area containing the sample inlet of each container.

## Description

The present invention relates to a device for transporting biological and similar samples, such as diagnostic samples, vaccines and pharmaceutical products.

More particularly, it relates to a device that is particularly suitable for the refrigerated or frozen transport of said samples, which are generally contained in test tubes.

Known devices of this type are made up of a number of receptacles in the form of test tubes situated inside an outer casing, which is generally parallelepiped-shaped. The space between the inner compartments and the outer casing is occupied by a substance of high thermal power which is resistant to the rise in temperature inside the receptacles due to heat exchange with the environment. The inner compartments and the outer casing are produced in a single piece of mouldable material, and the outer casing has an orifice for the injection of said material of high thermal power, which is sealed after the outer casing has been filled with said material. For its use, the device has been previously frozen, generally at temperatures of approximately -15°C for the transport of samples referred to as ' refrigerated' or at temperatures of approximately -30°C for those referred to as' frozen'. Once the device has been frozen, the test tubes are fitted together in the receptacles and said receptacles are sealed with the corresponding caps. The samples can thus be transported while keeping them in a refrigerated or frozen state.

Said devices known up to now have a number of drawbacks related to their construction. Firstly, these devices have to be produced in materials that are rigid at low temperatures, owing to the freezing and thawing forces that the test tube receptacles have to withstand, said receptacles, when in use, being surrounded by a substance that contracts and expands with the changes in temperature. This construction has the drawback of the outer casing therefore being excessively rigid, and therefore having little resistance to accidental impacts that may occur during transport of the samples. Another problem is that it is very difficult, if not impossible, to achieve proper sterilisation of the device after use, since there are regions that are difficult to access. Another drawback of the devices known at present is that, owing to their construction, the orientation of the device has to be monitored during freezing to ensure that the forces caused during freezing do not damage the device.

The present invention relates to a device for the transport of biological samples that does not have the drawbacks of the aforementioned known devices.

More specifically, to overcome the aforementioned drawbacks, the present invention consists of a device for the transport of biological and similar samples, of the type that comprises at least a receptacle suitable for the introduction therein of test tubes, an outer casing and a closure system for said receptacle or receptacles. The space between the receptacle or receptacles and said outer casing may be filled with a material that is resistant to the rise in temperature inside the receptacles due to heat exchange with the environment, characterised in that said receptacle or receptacles are formed as independent parts of the outer casing, each receptacle and the outer casing being tightly connected by the sample intake region of each receptacle.

By means of this construction, many advantages are achieved compared with the previously known devices. In particular, cleaning after use is not now a problem, since, as the receptacles are made up of independent parts, it is possible to disassemble them as required to disinfect the device. In addition, manufacture of the independent parts is technically and economically advantageous compared with that of devices of the known type. By using the device according to the invention, it is also possible to design the receptacles and the casing with different wall thicknesses and different materials, so that each element is better suited to its function. Thus, the receptacle or receptacles can be made more rigid and the outer casing more flexible, thus ensuring that any expansion of the substance situated in the space between the casing and the receptacles does not damage the sample. Moreover, the flexibility of the casing makes it more resistant to blows or impacts.

In preferred embodiments, the casing has at least a pair of depressed regions in its outer surface, situated facing each other on opposing walls of the casing between successive receptacles.

It is thus possible to produce a casing that combines high flexibility, which ensures that it is not broken when struck, with sufficient structural strength adequately to protect the receptacles, which are more fragile than the casing.

In another preferred embodiment, the intakes of each receptacle can be closed by a screw cap equipped with a seal to improve the tightness of the closure, and the seal makes contact with respective projections situated on the upper portion of the part or parts forming the receptacle or receptacles. The possibility of egress of the sample and cross-contamination of samples is thus avoided. The seal also increases the heat resistance of the device at the precise point where it is least heat resistant, in other words, heat exchange is made more difficult at the point where the device has the least favourable temperature conservation characteristics.

Finally, the present invention relates to a device for transporting biological samples which is cheaper and simpler to manufacture than currently known devices, without the aforementioned drawbacks, the design of which makes it easier to comply with the strictest transport regulations.

It will be better understood using the accompanying drawings of a preferred embodiment of the present invention given as an explanatory but not limiting example.
Fig. 1 is a perspective view of a device according to the present invention, with a cap unscrewed to reveal a test tube which is equipped with a cap and is being introduced into one of the receptacles of the device.
Fig. 2 is a perspective view partly in section through the plane II-II of Fig. 1.
Fig. 3 is a perspective view of a section through the mid-plane of the device of Figs. 1 and 2.
Fig. 4 is a front elevation of a device according to the present invention.
Fig. 5 is a front elevation and partial section of the part forming the receptacle according to the present invention.
Fig. 6 is a detailed view of the upper portion of the part of the receptacle shown in Fig. 5.
Fig. 7 is a perspective view partly in section of a cap with a seal for use as a cap for a receptacle of the device according to the present invention.

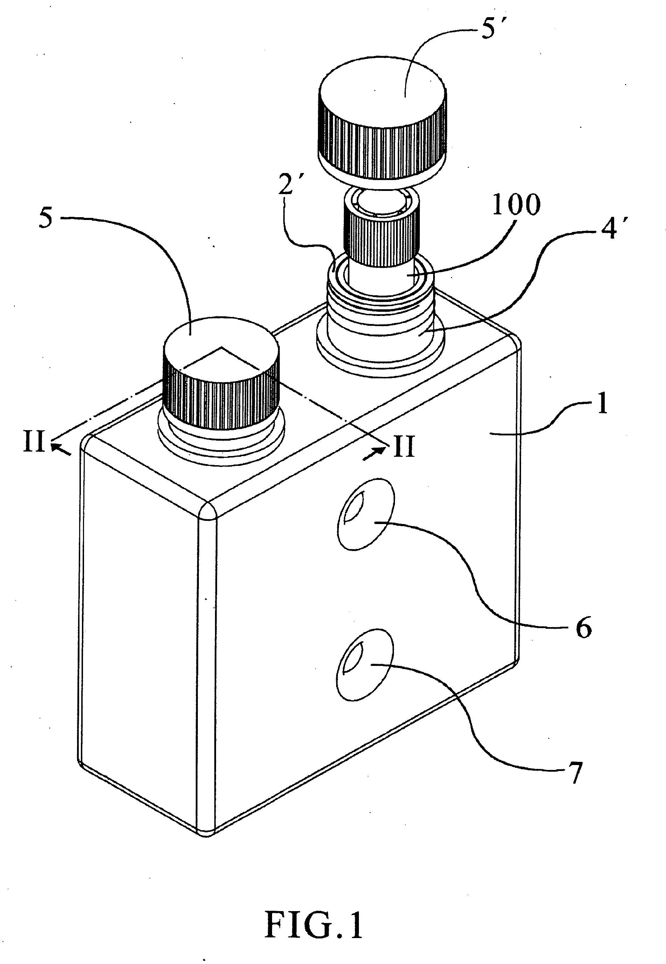

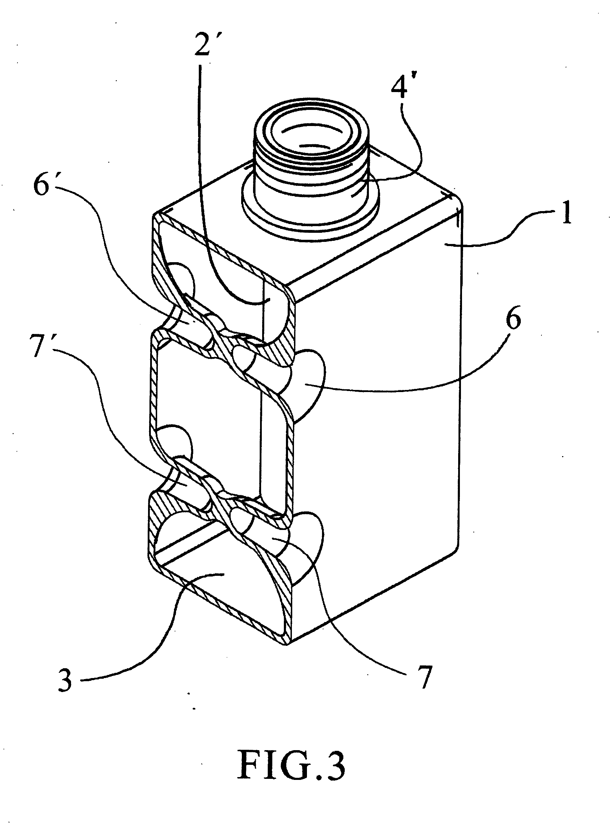

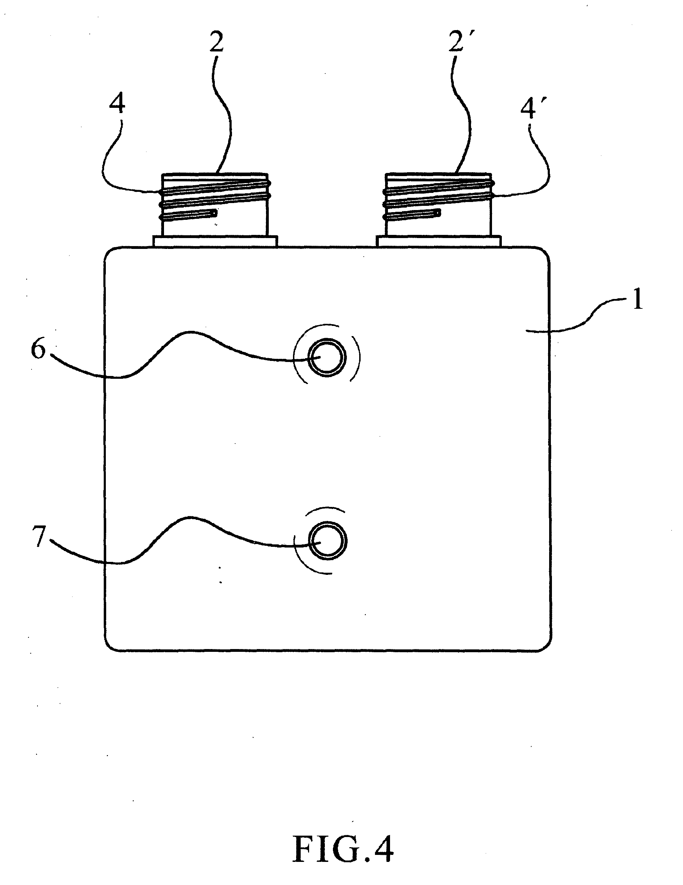

As can be seen in Figs. 1 to 4, the device for transporting biological and similar samples according to the present invention is made up of a variable number of receptacles 2, 2' suitable for the introduction therein of test tubes 100, an outer casing 1, which is of generally parallelepiped form in the example shown, and a closure system such as the screw caps 5, 5' for example, which may include a seal 8. The space 3 between the casing 1 and the receptacles 2, 2' is generally filled with a material of high thermal power in order to resist the rise in temperature inside the receptacles 2, 2'. In a manner characteristic of the present invention, the receptacles 2, 2' and the casing 1 are independent parts, and this allows them to be produced in different materials and/or thicknesses, depending on the different functions and mechanical stresses thereof. Thus for example, a flexible casing 1 may be designed to resist the blows or impacts that occur during transport, while the receptacles 2, 2' may be tougher, to ensure that an expansion of the material contained in the space 3 does not damage the transported sample. In the example shown, the sealed joint between the casing 1 and the receptacles 2, 2' is achieved by an interference fit, and the receptacles 2, 2' are therefore pressed into corresponding necks 4, 4'. In this way a sealed joint is obtained which allows the different parts making up the device to be disassembled, so that it is possible to access easily all regions of the device to clean and sterilise it, in particular the inner surface of the receptacles. Nevertheless, it should be stated that other means of obtaining a tight closure between the different parts, 1,2,2' making up the device are also possible.

In the example shown, it can be seen that the casing 1 has, on its outer surface, pairs of depressed regions 6, 6'; 7, 7' facing each other on opposite walls of the casing 1 in the region between the receptacles 2, 2'. These depressed regions give the casing 1 dimensional stability. Thus, the casing 1 may be chosen so that it is very flexible in order to improve resistance to blows which might occur during transport of the samples contained in the receptacles 2,2' of the device, without, however, said flexibility involving excessive dimensional instability of the container which could damage receptacles 2, 2' produced from a more rigid material, or could involve an increase in the pressure of the material contained in the space 3, endangering the required tightness in the joint between the casing 1 and the receptacles 2, 2'. In the example shown, each pair of depressed regions 6, 6'; 7, 7' is in physical contact. However, an embodiment is also possible in which the depressed regions 6, 6'; 7, 7' do not come into mutual contact. Moreover, the outer casing 1 in the example has rounded corners, and this improves the resistance of the device to any blows or impacts occurring during transport.

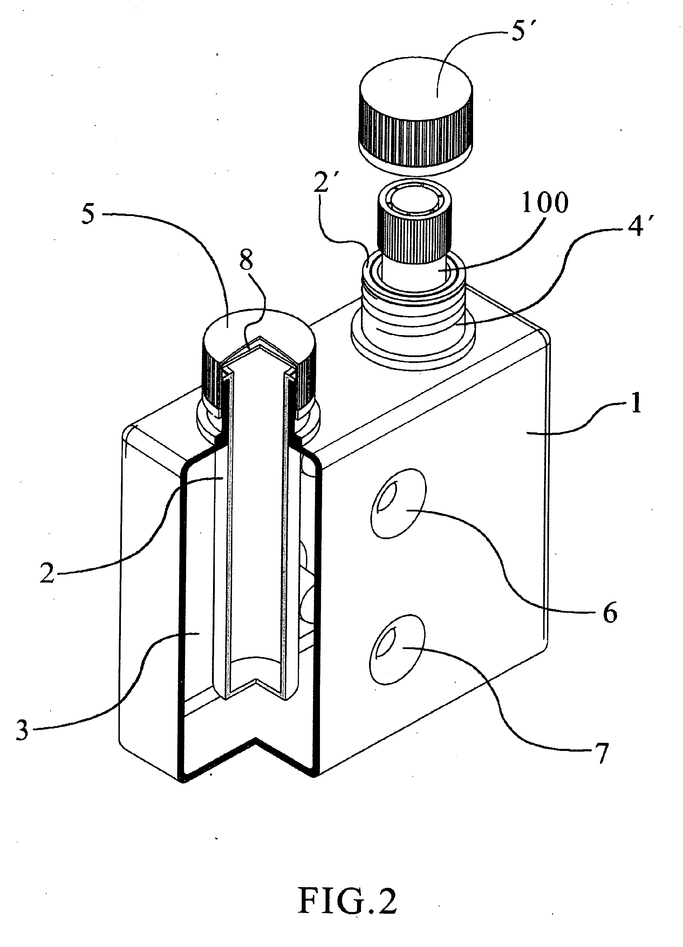

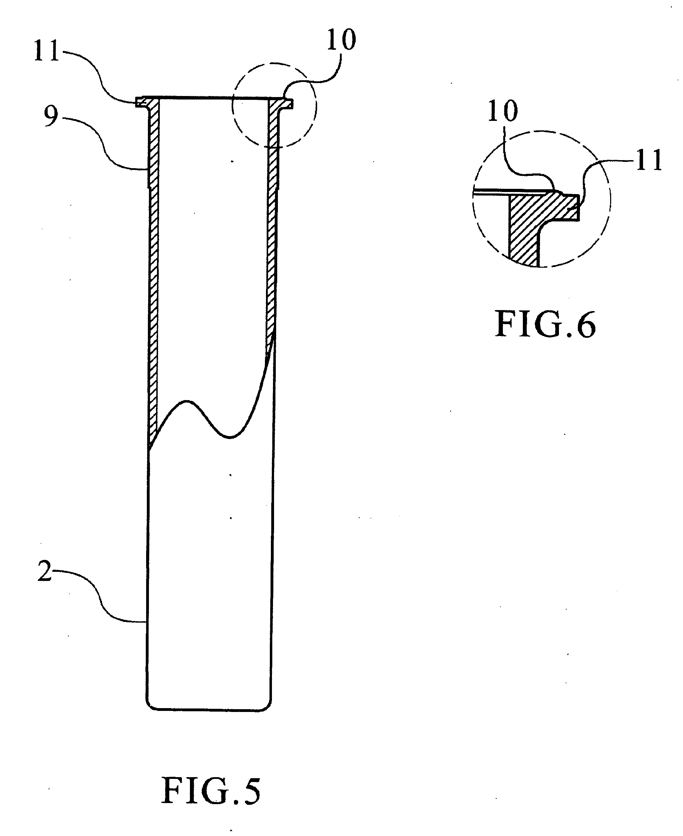

Fig. 6 shows an example of a receptacle 2 which has a generally cylindrical shape, having in its upper portion a flange 11 and a region of greater external diameter 9 to achieve a tight interference fit with the corresponding neck of the casing. The diameter of the receptacle may be chosen to accommodate comfortably a standard-size test tube or may be made larger to accommodate an absorbent material surrounding the sample. As can be seen, the base of the receptacle 2 is planar. This allows, for example, the introduction of test tubes (such as the test tube 100 shown in Figs. 1 and 2) upside down so that said test tubes rest on their upper cap.

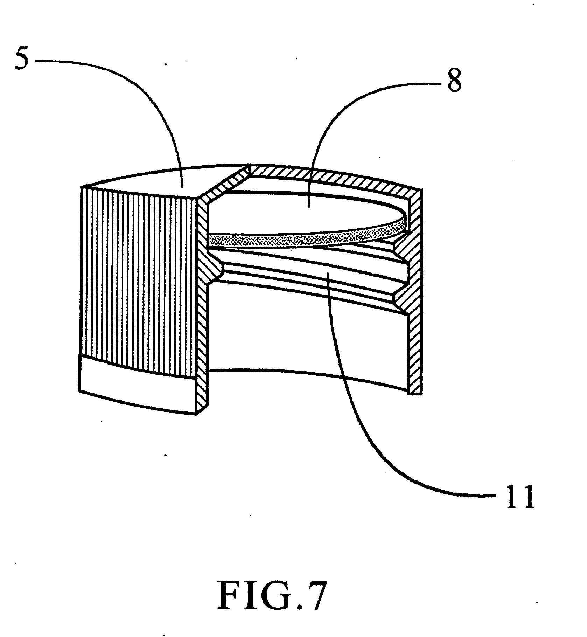

In addition, as shown in the detail in Fig. 6, the flange 11 also has a projection 10 on its upper face to make contact with a seal situated in the closure cap, thus improving the tightness of the closure of the device, and helping to maintain the temperature of the sample. An embodiment of a cap 5 is shown in Fig. 7 in which it can be seen that the cap 5 has a thread 11 and a seal, e.g. as a planar seal 8 of a known type.

Many variants of the example shown exist and will be obvious to persons skilled in the art. Thus, for example, the general form of the device, the number and arrangement of the receptacles and the depressed regions of the outer casing may vary. The closure system of the receptacles may also vary, for example a pressure cap or other known device may be used.

## Claims

1. Device for the transport of biological and similar samples of the type that comprises at least a receptacle suitable for the introduction therein of test tubes, an outer casing and a closure system for said receptacle or receptacles, wherein the space between said receptacle or receptacles and said outer casing may be filled with a material that is resistant to the rise in temperature inside the receptacles due to heat exchange with the environment, **characterised in that** said receptacle or receptacles are formed as independent parts of the outer casing, each receptacle and the outer casing being tightly connected by the sample intake region of each receptacle.

2. Device according to claim 1, **characterised in that** the casing has at least a pair of depressed regions in its outer surface, situated facing each other on opposing walls of the casing between successive receptacles.

3. Device according to claim 2, **characterised in that** at least two of said depressed regions of the casing, opposite each other, come into mutual contact.

4. Device according to any one of claims 1 to 3, **characterised in that** the tight closure is produced by pressure between the part or parts forming the receptacle or receptacles and said casing.

5. Device according to any one of claims 1 to 4, **characterised in that** the casing and the part or parts forming the receptacle or receptacles are produced in different materials and/or thicknesses.

6. Device according to any one of claims 1 to 5, **characterised in that** the closure system of the receptacle or receptacles consists of a screw cap or screw caps equipped with a seal to improve the tightness of the closure, which make contact with respective projections situated on the upper portion of the part or parts forming the receptacle or receptacles.

7. Device according to any one of claims 1 to 6, **characterised in that** the casing is of generally parallelepiped shape with rounded corners.

8. Device according to any one of claims 1 to 7, **characterised in that** the base of the receptacle or receptacles is planar.
